# EUROPEAN PATENT APPLICATION

(11) **EP 1 854 472 A1**
(43) Date of publication of application: **14.11.2007**
(21) Application number: 06713173.0
(22) Date of filing: 07.02.2006
(51) Int. Cl.: A61K 38/00, A61K 38/28, A61K 39/385, A61K 47/02, A61P 3/10, A61P 37/06, A61P 37/08

(54) **SUPPORT FOR PROTEIN TRANSFER, PROTEIN TRANSFER AGENT USING THE SUPPORT, PROTEIN TRANSFER METHOD, CELL HAVING PROTEIN TRANSFERRED THEREINTO AND METHOD OF PRODUCING THE SAME**

(30) Priority: 09.02.2005 JP 2005033145
(71) Applicant: OSAKA UNIVERSITY, Suita-shi, Osaka 565-0871 (JP); Meiji Dairies Corporation, Koto-ku, Tokyo (JP); Osaka Industrial Promotion Organization, Chuo-ku, Osaka-shi Osaka 5410053 (JP)
(72) Inventor: YAGI, Kiyohito c/o OSAKA University, Osaka 565-0781 (JP); KAWASE, Masaya c/o OSAKA University, Osaka 565-0781 (JP); YAMADA, Masashi c/o Meiji Dairies Corporation, Tokyo 136-8908 (JP); SUZUKI, Hiroto c/o Meiji Dairies Corporation, Tokyo 136-8908 (JP); KINO, Kohsuke c/o Meiji Dairies Corporation, Tokyo 136-8908 (JP); OHYAMA, Yoshio c/o Meiji Dairies Corporation, Tokyo, 136-8908 (JP); OHTSUBO, Kazumitsu c/o Meiji Dairies Corporation, Tokyo 136-8908 (JP)
(74) Representative: Golding, Louise Ann
(86) International application number: PCT/JP2006/302030
(87) International publication number: WO 2006/085516

(57) **Abstract**

A protein introduction method with which protein can be introduced into cells with excellent safety is provided. A target protein is supported on a carrier for protein introduction that is made from a clay mineral, and by adding this to cells it is possible to introduce the target protein into the cells. The clay mineral is preferably a layered clay mineral, and as the clay mineral it is possible to use montmorillonite, vermiculite, and illite, for example.

## Description

### Technical Field

The present invention relates to carriers for protein introduction for introducing protein into a cell, and to protein introduction agents using said carriers. The present invention also relates to protein introduction methods that use said introduction agents, and to cells to which protein has been introduced and methods of producing the same.

### Background Art

In recent years, so-called gene therapy, in which a normal gene that corresponds to a defective gene or a mutant gene is introduced into a cell so as to effect treatment at the genetic level, has been put to practical use as a method for treating various diseases, including cancer and genetic diseases. With gene therapy, however, although the gene is introduced into a cell, whether or not a protein is reliably expressed and its function exhibited becomes an issue.

This has led to examination into methods for introducing a required protein itself directly into a target cell, in addition to gene therapy. One approach that has been researched is the use of a carrier that supports a target protein and is introduced into a cell, and because the carrier is to be introduced into a living organism, it is crucial that it is safe.

However, there are very few carriers with which it is possible to introduce a protein into a cell, and on top of this, the carriers such as polyethyleneimine that are well-known are toxic to living organisms and thus pose a safety issue, and in practice there are essentially no carriers that can be used with living organisms (for example, see Non-Patent Document 1).
Non-Patent Document 1: Zelphati, O. et al. J. Biol. Chem. 276, 35103-35110 (2001) Intracellular delivery of proteins with a new lipid-mediated delivery system.

### Disclosure of Invention

### Problem to be Solved by the Invention

Accordingly, it is an object of the invention to provide a novel carrier for protein introduction, protein introduction agent, protein introduction method, and protein-introduced cells and method for producing the same, that have excellent safety.

### Means for Solving Problem

To achieve this object, the carrier for protein introduction of the invention is a carrier for introducing protein to a cell, and contains a clay mineral. The protein introduction agent of the invention is an introduction agent that includes the carrier for protein introduction of the invention and a protein that is supported on this.

Further, the protein introduction method of the invention is a method of bringing the protein introduction agent of the invention into contact with cells in order to introduce the protein into the cells, and through this method, the cells of the invention to which an exogenous protein has been introduced, are produced.

### Effects of the Invention

The inventors performed keen investigations with the aim of developing a novel carrier for protein introduction with a high degree of safety for cells in fields such as clinical medicine. As a result, they found that it is possible to easily introduce a protein into a cell by using the clay mineral as the carrier for protein introduction. The "clay mineral" ordinarily refers to the silicate minerals that are found in clay, and conventionally these have been used widely as components of cosmetics, as the base material of compression agents or antacids in the medical field, and as optical material and catalysts. However, as in the present invention, the fact that it is possible to use clay minerals as carriers for introducing protein into cells was first found by the present inventors.

According to the carrier for protein introduction of the invention, for example simply by blending the carrier and a protein to be introduced within a solution, it is possible to support the protein (it is possible to form a complex between the carrier and the protein), and moreover, by bringing the carrier on which the protein is supported into contact with a target cell, it is possible to introduce the protein into the cell. Thus, the act of introducing protein also is extremely simple. Also, the fact that clay minerals conventionally have been used widely in cosmetics and drugs, as described above, is sufficient proof that they are safe for living organisms, humans in particular. Consequently, the carrier for protein introduction of the invention, which includes such clay mineral with an excellent safety record, is widely applicable in and quite useful in fields such as clinical medicine. It should be noted that while the mechanism by which the protein introduction carrier of the invention introduces protein into cells is not clear, it is likely that the carrier for protein introduction is incorporated into cells while still supporting its protein, and then once within a cell releases its protein.

As discussed earlier, the protein introduction agent of the invention can be prepared very easily because by mixing the protein introduction carrier of the invention with a target protein it is possible to support that protein on the carrier. With the protein introduction method of the invention, which uses this protein introduction agent, it is possible to introduce a protein into a cell simply by bringing the introduction agent into contact with the cell, and thus the procedure also is extremely simple and has excellent safety.

The protein-introduced cells of the invention can be produced by the introduction method of the invention, which is extremely safe for cells, and thus it is for example possible to avoid the negative effects that are observed when a conventional carrier such as polyethyleneimine is used, for example. Therefore, the invention can be very safely adopted in treatment methods, for example, in which cells to which an exogenous protein has been introduced are brought into contact with the tissue cells of a lesion. It should be noted that "protein" as used in the invention is not limited literally to proteins, and also includes peptides or the like, as will be discussed later.

### Brief Description of Drawings

FIG. 1 is a graph that shows the β-galactosidase activity in the protein-introduced cells in a working example of the invention.
FIG. 2 is a graph that shows the β-galactosidase activity in the protein-introduced cells in another working example of the invention.
FIG. 3 is a graph that shows the degree of adsorption of the protein to the protein introduction carrier, in a yet further working example of the invention, where FIG. 3(A) shows the amount of protein in the supernatant fraction, and FIG. 3(B) shows the rate of adsorption of the protein to the protein introduction carrier.
FIG. 4 is a graph that shows the amount of OVA specific IgG in mouse serum in a case where OVA has been introduced by the protein introduction carrier, in a yet further working example of the invention.
FIG. 5 is a Western blot electrophoresis photograph that shows whether or not OVA is present in cells in a case where midkine has been introduced by the protein introduction carrier, in a yet further working example of the invention.
FIG. 6 is a Western blot electrophoresis photograph that shows whether or not pleiotrophin (PTN) is present in cells in a case where PTN has been introduced by the protein introduction carrier, in a yet further working example of the invention.
FIG. 7 is a Western blot electrophoresis photograph that shows whether or not insulin is present in cells in a case where insulin has been introduced by the protein introduction carrier, in a yet further working example of the invention.

### Best Mode for Carrying Out the Invention

### First Embodiment

### Carrier for Protein Introduction

In the carrier for protein introduction of the invention, there are no particular limitations regarding the clay mineral, and although the mechanism is unclear, it is possible to support a protein with the clay mineral. An example of the clay mineral is a layered clay mineral. Clay mineral with a layered structure ordinarily takes on a structure in which ions or water are sandwiched between its layers.

It is preferable that the substance between the layers of the layered clay mineral includes an exchangeable cation, for example. If the interlayer substance includes an exchangeable cation, then ordinarily the cation is bonded with hydroxides (-OH) on the inner surface of the layer. It should be noted that clay mineral that has an exchangeable ion as the substance between its layers hereinafter will be referred to as "exchangeable cation-type" clay mineral.

With such clay mineral, it is for example possible to release (free) protein under neutral or alkaline conditions but not release the protein under acidic conditions. The clay mineral thus is useful when a protein is to be selectively released in accordance with the conditions.

Examples of the exchangeable cation include sodium ion, ammonium ion, and tertiary ammonium ion. Of these, sodium ion is preferable because natural clay mineral often contains sodium, but tertiary ammonium ion with its large three-dimensional structure also is preferable. Examples of tertiary ammonium ions include tetramethylammonium chloride ion and benzyltrimethylammonium chloride ion.

In addition to these, the exchangeable cation also can be the cation of various amino acids such as a-amino acids or β-amino acids, an amine compound such as dopamine, or an amide compound such as acrylamide, a cationic surfactant such as alkylammonium, alkyltrimethylammonium, tetramethylphosphonium, and alkylpyridinium, or the cation of a cationic metal complex such as ruthenium tetraammonium and tris(phenanthroline)rhodium. As the cationic metal complex it is also possible to use an ammonium complex of ruthenium, for example. It also may be methyl viologen.

There are no particular limitations regarding the layered clay mineral of the invention, and for example, it is possible to use a crystalline clay mineral from the total of eight groups of kaolinite, pyrophyllite-talc, smectite, vermiculite, mica, brittle mica, chlorite, and sepiolite-paligoskite. Of these crystalline minerals, the silicate layer type is 1:1 in the kaolinite group and 2:1 in the remaining seven groups. Examples of clay mineral that belongs to the kaolinite group, in which there is no layer charge, include kaolinite, dickite, halloysite, nacrite, chrysotile, and lizardite, and examples of clay minerals that belong to the pyrophyllite-talc group, which also has no layer charge, include pyrophyllite and talc. Examples of the smectite group, which has a layer charge, include montmorillonite, beidellite, nontrolite, saponite, hectorite, and stevensite, examples of the vermiculite group include di-vermiculite and tri-vermiculite, examples of the mica group include muscovite, paragonite, illite, phlogopite, biotite, and lepidolite, examples of brittle mica include margarite and clintonite, and examples of chlorite include donpasite, sudoite, cookeite, clinochlore, and chamosite. Examples of the sepiolite-paligoskite group, which has no layer charge, include sepiolite and paligoskite. Of these, a crystalline clay mineral that has a layer charge is preferable, and the smectite group, the vermiculite group, and the mica group are more preferable. Specifically, montmorillonite, vermiculite, and illite are preferable, and montmorillonite is particularly preferable.

In addition to these layered clay minerals, it is also possible to use a noncrystalline clay mineral such as imogolite, allophane, and hisingerite. There is no limitation to natural clay minerals, and it is also possible to use synthetic clay minerals as well.

The clay mineral can be extracted from natural clay, but it is also possible use various commercially available products. As montmorillonite it is for example possible to use bentonite, of which montmorillonite is a principal component. Bentonite readily can be purchased as a commercial product that conforms to "bentonite" as listed in the Pharmacopoeia of Japan, and because these have been proven to not be harmful to living organisms, they are extremely useful as the raw material of the carrier for protein introduction of the invention. Examples of such commercially available bentonite include Bentonite (trade name) and Kunipia F (trade name) (both made by Kunimine Industries). Examples of saponite include Sumecton SA (trade name) (made by Kunimine Industries). It is also possible to obtain various clay minerals from The Clay Science Society of Japan. It should be noted that the carrier for protein introduction of the invention can include single clay minerals, a mixture of a plurality of clay minerals, and components other than clay minerals, and there are no limitations regarding the purity of clay mineral.

The clay mineral can be purified clay mineral from which various impurities contained therein or adhered thereto have been removed. Examples of impurities include organic compounds in soil (various amino acids and lignin), iron, potassium, calcium, and magnesium.

In the case of a purified clay mineral, the purity by ordinary x-ray diffraction or elemental analysis is for example at least 85%, preferably at least 95%, and particularly preferably at least 98%. As is general practice, the purity by x-ray diffraction can be determined from the intensity ratio of the diffraction line, in which the diffraction pattern is compared against a reference sample.

There are no particular restrictions regarding the method for removing these impurities, and for example, if an organic compound is to be removed then it is possible to adopt the following method.

The removal of organic compounds can be carried out by for example adding a hydrogen peroxide solution to the clay mineral and dispersing the clay mineral therein, and then heating that dispersion. The concentration of the hydrogen peroxide solution is for example 3 to 15 wt%, preferably 3 to 10 wt%, and more preferably 5 to 7 wt%. The amount of hydrogen peroxide solution that is added per 1 g clay mineral is for example 10 to 30 mL, preferably 15 to 30 mL, and more preferably 15 to 20 mL. The heating condition is for example a temperature of 25 to 50°C, preferably 25 to 40°C, and more preferably 30 to 40°C. There are no particular restrictions regarding the heating time, and for example it is preferable to perform heating until bubbles (oxygen) due to heating disappear, and it is preferable to conduct further heating after the formation of bubbles has stopped in order to remove residual hydrogen peroxide. It should be noted that there is no limitation to these conditions.

Through such process for removing organic compounds, ordinarily it is possible to obtain clay mineral with 85 to 100% purity. The organic compound content of the clay mineral that is used is for example 5% or less, preferably 2%, and more preferably 1% or less.

If a clay mineral that has been subjected to the process for removing organic compounds is to serve as the carrier for protein introduction, then it is possible to recover clay mineral from the dispersion by filtration or centrifugation, for example, and then wash this with a solvent such as water, a buffer solution, or saline solution. It should be noted that the substance between the layers of the clay mineral that has been subjected to such process for removing organic compounds is the same as in the clay mineral prior to the process that is used as a raw material, and examples include various exchangeable cations such as sodium ion as well as potassium ion, calcium ion, and magnesium ion.

In order to remove iron that is adhered to or included in the clay mineral, it is also possible to subject the clay mineral to deironization as described below in lieu of or in addition to the process for removing organic compounds. The deironization can be performed by the method shown below.

The dispersion solution of the clay mineral that was treated with the hydrogen peroxide solution is heated and then a sodium citrate aqueous solution and a sodium hydrogen carbonate aqueous solution are added thereto and this is sufficiently agitated. A sodium hydrosulfite solution aqueous solution also is added to this mixture and agitated, and the mixture is left undisturbed. To have the clay mineral after deironization serve as a carrier for protein introduction, it is sufficient to wash this mixture with various solvents like in the case of the process for removing organic compounds and then use the resulting material.

In this method, the amount of the substances that is added per 1 g clay mineral is for example a range of sodium citrate 50 to 70 mmol, sodium hydrogen carbonate 15 to 30 mmol, and hydrosulfite 20 to 35 mmol, and preferably the range is sodium citrate 55 to 65 mmol, sodium hydrogen carbonate 22 to 28 mmol, and hydrosulfite 26 to 29 mmol.

It is also possible to induce the clay mineral after the process for removing organic compounds or after deironization to take on any one of various exchangeable cationic forms through an ion-exchange reaction. If, prior to processing, the clay mineral contains an exchangeable cation such as sodium between its layers, then it is possible to execute this induction. Specifically, an example of inducing the clay material to an exchangeable sodium form is shown below.

In a case where the clay mineral is to be induced to take on an exchangeable sodium form, then, for example, it is possible to add a clay mineral that has been subjected to organic compound removal or deironization to, and disperse in, a sodium salt saturated aqueous solution such as sodium chloride saturated aqueous solution, and then recover the clay mineral by filtration or centrifugation. The addition of the sodium salt (such as sodium chloride) and the recovery of the clay mineral may be performed once, but in order to sufficiently induce a transition to a sodium form, preferably these are performed two or more times, and more preferably 3 to 5 times. The amount of sodium chloride saturated aqueous solution that is added per 1 g clay mineral is for example in the range of 30 to 80 mL, preferably 40 to 60 mL, and more preferably 50 to 60 mL. There are no limitations regarding the precipitation of the clay mineral in the sodium chloride saturated aqueous solution, but preferably this takes at least 10 seconds, and more preferably between 30 and 60 seconds.

The solvent that is used to induce a transition to an exchangeable sodium form is not limited to a sodium chloride aqueous solution, and in addition to this it is also possible to use an aqueous solution that contains a sodium salt such as sodium bromide or sodium iodide. There are no particular restrictions regarding the concentration of the sodium salt in these solutions, but preferably the solutions are saturated solutions as discussed above.

To more completely induce a transition to a sodium form, it is possible to perform induction with an aqueous solution of a sodium salt such as sodium citrate, sodium hydrogen carbonate, sodium acetate, sodium chloride, sodium bromide, sodium iodide, and sodium azide, or with an aqueous solution that contains two or more sodium salts, in place of or in addition to induction with the sodium chloride saturated aqueous solution.

On the other hand, if the clay material is to be induced to an exchangeable ammonium form, then it is possible to perform the same process as in the method of induction to a sodium form, with a solution that includes ammonia or an ammonium salt such as ammonium chloride, ammonium bromide, ammonium iodide, or ammonium acetate. In the case of a tertiary ammonium form, it is possible to use a solution that contains a tertiary ammonium salt such as tetramethylammonium chloride or benzyltrimethylammonium chloride. It is also possible to modify the mineral function through pillarization using a cationic surfactant such as those discussed above, or through substitution with an amine compound, an amide compound, or a cationic metal complex, for example, such as those discussed above.

It is also possible to use clay mineral that has been induced to take on an exchangeable cation form as the carrier for protein introduction after dialysis.

The various clay minerals discussed above can be used as a carrier for protein introduction as they are, dispersed in solution, for example, and it is also possible to dry them by freeze drying, for example, and then use the dried product as a carrier for protein introduction. This is because dry clay mineral easily can be adjusted to a desired concentration when preparing the protein introduction agent and is in the form of a dispersion, which is discussed later. It should be noted that the carrier for protein introduction of the invention is not limited to protein only, and it also can introduce peptides.

### Second Embodimnent

### Protein Introduction Agent

The protein introduction agent (which also may be referred to as protein complex) of the invention includes the carrier for protein introduction of the invention, which as discussed earlier includes clay mineral, and a protein that is supported on this. It should be noted that "protein" as used in this invention includes not only generally discussed protein that is made up of only amino acids but also includes peptides, glycoproteins, and mucin, for example, and physiologically allowable salts of those as well (in this invention, these shall be referred to as "protein"). Examples of physiologically allowable salts include inorganic acid-addition salts such as hydrochloride, sulfate, and phosphate, organic acid salts such as acetate, proprionate, citrate, tartarate, and malate, and other salts such as sodium salts, potassium salts, and calcium salts.

The blend ratio (mass ratio A:B) of the protein (A) and the clay mineral (B) in the protein introduction agent is for example in the range of 1:0.1 to 5, preferably in the range of 1:0.5 to 4, more preferably in the range of 1:0.5 to 3, and particularly preferably in the range of 1:1 to 2.

There are no particular limitations regarding the protein that is supported by the carrier for protein introduction, and various proteins may be used both singly and as mixture of a plurality of proteins. There are absolutely no limitations regarding the characteristics of the protein, and the protein can be sufficiently introduced into a cell regardless of whether it is a peptide, which has a low molecular weight, or a protein, which as a high molecular weight, for example. There are no limitations regarding the molecular weight, and one specific example is 300 Da to about 500 kDa (of course also including larger proteins and smaller peptides), preferably in the range of 1 kDa to 500 kDa, and more preferably in the range of 5 kDa to 500 kDa. These can be adequately introduced into a cell. There also is no limitation regarding the type of clay minerals with which the protein is combined, and for example, it is possible to choose the clay mineral type in accordance with the protein type.

The type of protein can for example be determined suitably in accordance with the function to be given to the cell, etc., and for example, in the medical field, examples include proteins that serve as agents for treating, agents for preventing, and agents for inhibiting a disease. One specific example is a protein that is used for hyposensitization treatment. For example, illustrative examples include making an allergen, etc., of Japanese cedar pollen supported by the carrier in order to treat hay fever, or making a target protein (protein or fragment thereof responsible for the allergy) such as egg albumin (ovalbumin) supported by the carrier in order to treat a food allergy, and then this is introduced into the cell. In order to treat a disease due to an enzyme deficiency, then it is also possible to make a deficient enzyme supported by the carrier, and in order to treat lactose intolerance it is possible to make β- galactosidase supported by the carrier, and then introduce them into the cell. For diabetes, it is possible to make insulin supported by the carrier and then introduce this into the cell.

There are no particular limitations regarding the form of the protein introduction agent as long as it includes the carrier for protein introduction and the protein that is supported on that carrier, and for example, the form can be a dispersion in which the protein introduction agent is dispersed, or the protein introduction agent can be in the form of a freeze-dried product. The protein introduction agent may also be in the form of a frozen dispersion, and in this case, it can be defrosted when used. As necessary it is also possible to add pharmaceutical additives such as excipients, linkers, disintegrators, stabilizers, preservatives, and buffers, or food additives such as sweetener, perfume, food preservatives, and stabilizers.

Next, an example of the method of preparing the protein introduction agent of the invention is shown. First, a protein dispersion that contains a target protein, and a clay mineral dispersion that includes the clay mineral (carrier for protein introduction), are prepared.

There are no particular limitations regarding the concentration of protein in the protein dispersion, and for example, it is in the range of 0.1 to 1000 µg/mL, preferably in the range of 25 to 400 µg/mL, and particularly preferably in the range of 50 to 200 µg/mL. There are no particular limitations regarding the dispersion solvent of the dispersion solution, and it may for example be distilled water, saline solution, or PBS (phosphate buffered saline). If the invention is to be used to introduce protein to cultured cells as described later, then in addition to these it is also possible to use a liquid medium, for example. It should be noted that the dispersion solvent preferably has been sterilized in order to prevent contamination. There are no particular limitations regarding the pH of the protein dispersion, and for example it can be near neutral pH, and preferably is in the range of 5.5 to 7.5.

There are no particular limitations regarding the concentration of the clay mineral in the clay mineral dispersion, and for example it is in the range of 0.1 to 1000 µg/mL, preferably in the range of 25 to 400 µg/mL, and particularly preferably in the range of 50 to 200 µg/mL. The same dispersion solvent can be used as the solvent for this dispersion. The pH of the clay mineral dispersion is the same as the protein dispersion, and there are no particular limitations regarding it.

It is possible to prepare a protein introduction agent by mixing the protein dispersion and the clay mineral dispersion. By blending the protein and the clay mineral in solution in this way, it is possible to form a complex in which the protein is supported on the clay mineral. It should be noted that the blend ratio (mass ratio A:B) of the protein (A) and the clay mineral (B) in the protein introduction agent that is obtained by mixing the protein dispersion and the clay mineral dispersion is the same as discussed above, for example.

The mixture (protein introduction agent) obtained by blending the protein dispersion and the clay mineral dispersion ordinarily is aimed at cells or living organisms, and thus, although there are no particular limitations to its pH, for example, the pH can be set to near a neutral pH (for example, a pH of approximately 6.5 to 7.5). As discussed above as well, there are no limitations also in a case where the clay mineral contains an exchangeable ion between its layers, and for example, the pH can be adjusted to near a neutral pH. It should be noted that for example, even if the agent is placed in acidic conditions while holding the protein, the protein can be introduced without being affected by the pH. Thus, there is no impact on the introduction of the protein itself by way of the stomach, which has highly acidic conditions, and the agent can be considered to be suited for oral administration as discussed later.

It is possible to use the mixture as the protein introduction agent immediately after blending the protein dispersion and the clay mineral dispersion, but in order for the protein to be sufficiently supported on the clay mineral, it is preferable that the mixture is incubated after blending. There are no particular restrictions regarding the temperature conditions of the incubation, and ordinarily, incubation can be carried out at room temperature, and there are no particular restrictions regarding the time of the incubation either.

The protein introduction agent can be used as it is in dispersion form, or it may be frozen until used as discussed above. If the agent is to be dried, then, for example, it is possible to adopt a method such as freeze drying or spray drying, and then when the agent is to be used it can be dispersed within a dispersion solvent like those discussed above.

The clay mineral of the invention has been known to be ingested as a natural treatment, for example, and may be contained in preparations that are to be taken orally. Because it has been sufficiently proven to be safe for living organisms, the protein introduction agent of the invention is extremely useful as a drug, functional food, or food additive.

The protein introduction method of the invention, in which the protein introduction agent is brought into contact with a cell and the protein is introduced, is described by specific examples in vitro and in vivo in a third embodiment and a fourth embodiment, respectively. This method allows cells to which exogenous protein has been introduced to be produced. It should be noted that there is not a limitation to these implementations.

### Third Embodiment

### Protein Introduction Method in vitro

This embodiment describes an example of the introduction method in which, in vitro, the protein introduction agent is brought into contact with cultured cells and a target protein is introduced into the cells. In this method, it is possible to add the protein introduction agent to the cultured cells to which the target protein is to be introduced and then carry out the incubation.

It is preferable that the cultured cells are pre-incubated for a fixed period of time in a liquid culture medium that corresponds to the cell type, and then the protein introduction agent is added and the cells are incubated further. By performing a pre-incubation prior to adding the introduction agent, it is for example possible to introduce the protein smoothly into the cultured cells with the introduction agent, and by incubating for a fixed period of time after the protein has been added, it is possible to introduce the protein more efficiently.

The culture medium type can be determined suitably in agreement with the cells as mentioned above, and although there are no particular limitations, it is for example preferable that the medium contain serum because it can stabilize the cell growth environment.

There are no particular limitations regarding the ratio with which the protein introduction agent is added to the cultured cells, but for example, it is preferable for the protein introduction agent to be added such that the clay mineral is added at a ratio in the range of 0.01 to 100 µg per 1×10⁶ cells, more preferably such that the clay mineral is added at a ratio of 0.1 to 50 µg, and particularly preferably 0.2 to 25 µg. It is preferable for the protein introduction agent to be added such that the protein is added at a ratio in the range of 0.01 to 100 µg per 1×10⁶ cells, more preferably such that the protein is added at a ratio of 0.1 to 50 µg, and particularly preferably 0.2 to 25 µg.

There are no particular limitations regarding the cultured cells to which the target protein is to be introduced, and for example, the method of the invention can be adopted for various cells, including cells of the small intestine, nasal mucosa, skin tissue, subcutaneous tissue, bone tissue, cartilage tissue, and periodontal cells. In addition to human cells and animal cells other than humans (mammalian cells), it is also possible to adopt the method of the invention for the cells of all species, including microorganisms, fish, reptiles, amphibians, birds, and insects. As mentioned previously, there are no particular limitations regarding the cell culture, and it is possible to use a liquid medium that is known to the public that corresponds to the type of cell, and then carry out the culturing in accordance with culturing conditions that are public knowledge.

A specific example of the culture conditions is shown below, but because the culture conditions can be suitably chosen in accordance with the type of cultured cell as mentioned previously, there is no limitation to these conditions. For example, it is possible to pre-culture (pre-incubate) the cells prior to adding the protein introduction agent to the cultured cells, and the culture time is, for example, preferably 18 to 30 hours, more preferably 18 to 24 hours, and particularly preferably 22 to 24 hours. On the other hand, there are no particular limitations regarding the incubation time after addition of the protein introduction agent, and for example, it is sufficient for the protein to be introduced into the cells, and the incubation time is about 3 hours, for example, and although the upper limit is not particularly limited, the upper limit is about 48 hours. The culture temperature ordinarily is 37°C, and preferably the cells are cultured in the presence of 5% carbon dioxide.

The cells to which protein has been introduced that are obtained through the above method can be used for the following therapy. For example, in a case where a certain disease is caused by a gene deficiency, the protein that is coded for by the gene is introduced into the cultured cells through the introduction method of the invention. Then, the resulting protein-introduced cells are then administered to the part affected by that disease. By doing this, it is possible to alleviate or cure the symptoms of the disease due to the introduced protein in the cells that are administered. It should be noted that the method of administration can involve injection to the affected part or a surgical procedure of supporting the protein-introduced cells on a carrier and then implanting the carrier in the affected part, subcutaneously, or in the peritoneal cavity.

### Fourth Embodiment

Next, an example will be described in which the protein is introduced in vivo, that is, in which the cells are the cells of an organism and the protein introduction agent is administered to the organism in order to introduce the protein to organ or tissue, cells with the introduction agent. With this method, simply by administering the protein introduction agent orally or by administrating it to a target organ or tissue through a surgical procedure or injection, for example, it is possible to introduce a target protein to target cells in an organism.

If in the organism the protein is to be introduced into cells of the small intestine, then it is preferable that the protein introduction agent is administered orally. Oral administration is preferable because it is simple and the dose readily can be adjusted in accordance with the results of the treatment. Moreover, if the protein introduction agent of the invention is used, it is possible to introduce the target protein into cells of the small intestine selectively and reliably. It is conceivable that the protein can be introduced selectively to the small intestine in this manner for the following reason.

As mentioned above, the protein is supported on the clay mineral by mixing, etc., the clay mineral and the protein. A carrier for introduction that supports a protein in this manner ordinarily increases in charge when the pH is an acidic pH, and thus tightly supports the protein, whereas in neutral or alkaline conditions, its charge becomes smaller and the protein is freed and is released. Because the clay mineral has this mechanism, by orally administering the protein introduction agent, the protein passes through the acidic conditions of the stomach without being released and reaches the small intestine. Since the carrier is a clay mineral, it is not digested in the stomach. The small intestine has a pH that is near neural and its charge is smaller than in the stomach, and consequently the protein is released from the clay mineral of the protein introduction agent. Thus, if the carrier for protein introduction (protein introduction agent) of the invention is administered orally, the protein is not digested in the stomach and, unlike the case of intravenous injection, there is no problem of the protein being taken up by various cells and thus making it difficult to administer it selectively to the small intestine. In other words, with the invention, through simple oral administration it is possible easily to administer selectively the protein to the small intestine.

Selectively bringing a protein introduction agent into contact with the small intestine allows allergy treatment that utilizes gut immunity (hyposensitization treatment) to be achieved through simple oral administration. Protein's that cause various allergies are supported on a carrier for protein introduction in order to produce a protein introduction agent, and this is periodically administered orally to allergy patients. By doing this, the protein introduction agent is carried to the small intestine and the target protein is selectively released in the cells of the small intestine, and thus the phenomenon of immunological tolerance in the gut immunity occurs and the allergy symptoms are diminished. This method is an excellent therapeutic method that, compared to conventional hyposensitization therapy, does not require as many doses by injection and places less of a burden on the patient, for example. The cells of the small intestine ordinarily are sloughed away approximately every two weeks, and thus cells to which the protein has been introduced do not remain in an organism for a long period of time. Consequently, it can be said that adopting the present invention allows a prevention method and a therapeutic method with even greater safety to be provided.

The in vivo protein introduction method can be adopted for human organisms and non-human mammals alike. The dose of the protein introduction agent can be determined suitably according to the object of the agent and the type of the organism, and for example it is also possible for the ratio between the cells and the clay mineral or the protein to be the same as in the case of in vitro introduction that was described above.

Thus, with the in vivo protein introduction method described above it is possible to introduce protein very safely, and by administering the agent orally it is possible to introduce the protein selectively to the small intestine without performing a surgical procedure or an injection, and thus this method can be regarded as a useful method for clinical treatment.

### Fifth Embodiment

### Pharmaceutical Composition and Disease Prevention Method and Disease Treatment Method

The pharmaceutical composition of the invention contains protein as an agent for treating, an agent for preventing, or an agent for inhibiting, a disease, and is characterized in that it further includes the carrier for protein introduction of the invention (in order words, it is a pharmaceutical composition that includes the protein introduction agent of the invention). If a protein is the active component that serves as the therapeutic agent, protective agent, or inhibitory agent against a disease, then by administering the pharmaceutical composition of the invention, which includes the carrier for protein introduction of the invention, it easily is possible, and with excellent safety, to prevent and treat diseases in humans and in mammals other than humans. There are no limitations regarding the type of the protein, and it can be selected according to the disease, and for example, in the case of hyposensitization treatment, it is a protein such as a pollen or albumin, and in the case of diabetes, it is insulin, for example. There also are no restrictions regarding the manner in which the pharmaceutical composition is administered, and oral administration or non-oral administration such as injection can be selected according to the disease. The pharmaceutical composition of the invention also can be a protein-introduced cell as discussed above, and can be administered by surgically implanting it in an organism.

### Working Example 1

A clay mineral was used to introduce β-galactosidase to the epithelial cells of the small intestine in vitro.

### 1 Complexing the Protein and the Carrier for Protein Introduction

Using three clay minerals as the carriers for protein introduction, these being Bentonite (trade name) and Kunipia F (trade name), which include montmorillonite, and Sumecton SA (trade name), which includes saponite (all made by Kunimine Industries), these were dispersed to 1 mg/mL in PBS (-) (Ca²⁺, Mg²⁺-free phosphate buffer saline solution: pH 7.2) to prepare carrier dispersions. On the other hand, β-galactosidase from *Escherichia coli* (made by CAL BIOCHEM) was suspended in mili-Q water at 1 mg/mL to prepare a protein dispersion. Then, 5 µL carrier dispersion and 1 µL protein dispersion were added to and mixed in 100 µL DMEM (Dullbecco's Modified Eagle's Medium: serum-free). The mixture solutions were incubated at 25°C for one hour, producing protein introduction agents.

### 2. Introduction of Protein in vitro

As the cells, rat small intestine epithelial cells IEC-6 furnished from ATCC (ATCC-CRL1592) were used. First, 2.5 mL of the following liquid medium was added to a 12-well plate (made by Falcon), and 1×10⁵ small intestine epithelial cells were inoculated in each well and cultured at 37°C for 24 hours. It should be noted that during culturing, the carbon dioxide concentration was adjusted to 5% by a carbon dioxide incubator.

### (Liquid Medium Composition)

DMEM (Dullbecco's modified Eagle medium: made by Nissui Pharmaceutical Co., Ltd.)
10 wt% FBS (fetal bovine serum: Dainippon Sumitomo Pharma Co., Ltd.)

After culturing for 24 hours, the cultured cells were washed twice with the PBS (-), and 1 mL DMEM (serum-free) was added to each well. Further, 100 µL of the protein introduction agent was added to each well to a β-galactosidase concentration of 1 µg (905 mU)/well. This was incubated at 37°C for three hours, then 111 µL of the serum (FBS) was added to adjust the FBS concentration in the medium to 10 wt%, and this was incubated at 37°C for 24 hours.

As a control, cells to which the protein introduction agent was not added, and as a Comparative Example 1, cells to which only β-galactosidase was added, were cultured in the same manner. It should be noted that in Comparative Example 1, the protein dispersion (1 mg/mL) was diluted with 100 µL DMEM and added in place of the protein introduction agent.

After incubation, all cells were recovered. First, the cultured cells were washed twice with the PBS (-) and then 100µL PBS (-) was added and the cells were recovered with a Cell Scraper. The cells were then frozen at -80°C, then thawed at 37°C and centrifuged (14,500 rpm, 5 minutes), and the cell extract was recovered.

### 3. Verifying Protein Introduction

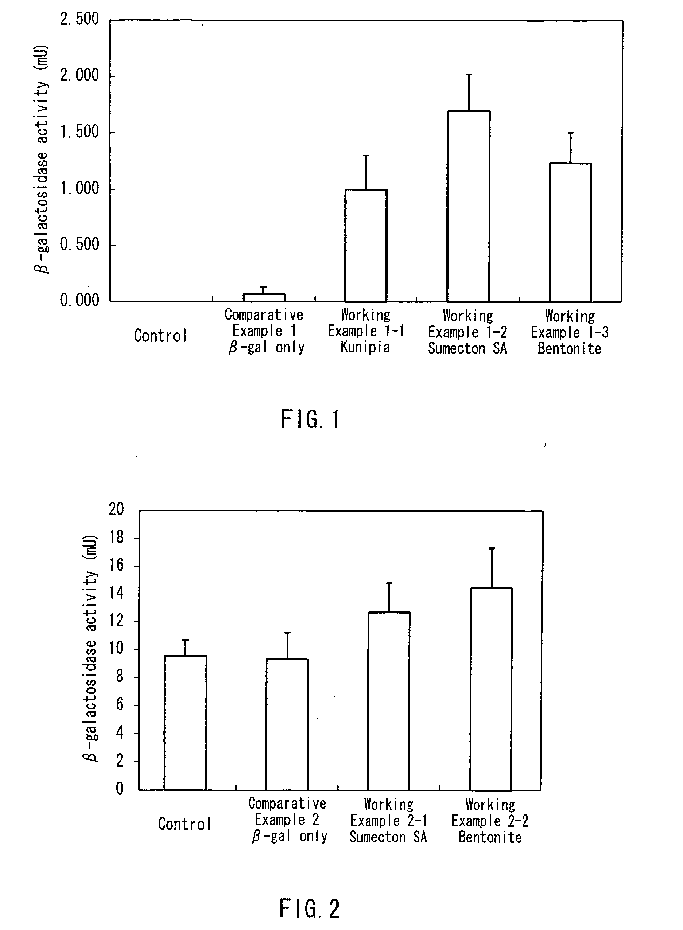

90 µL of the Z buffer, whose composition is shown below, 10 µL of the recovered cell extract, and 20 µL of a 4 mg/mL ONPG (o-nitrophenyl β-D-galactopyranoside) solution were added to a 96-well plate and incubated at 37°C for one hour, then 100 µL of a reaction stopping solution (1M Na₂CO₃) was added and the absorbance of the reaction solution at a 415 nm wavelength was measured. The β-galactosidase activity (mU) in the cells was calculated from the absorbance that is obtained and a pre-prepared calibration curve. The results are shown in FIG. 1. It should be noted that "β-galonly" in FIG. 1 indicates that in Comparative Example 1, only β-galactosidase was introduced (same in FIG. 2).

**(Z buffer)**

| | |
|---|---|
| Na₂HPO₄ | 851.76 mg |
| NaH₂PO₄ | 623.89 mg |
| KCl | 74.55 mg |
| MgSO₄ | 12.04 mg |
| 2-mercaptoethanol | 350.6 µL |
| mili-Q water | 1000 mL |

As shown in the drawings, Working Examples 1-1 (Kunipia), 1-2 (Sumecton SA), and 1-3 (Bentonite) exhibited much higher activity than in Comparative Example 1. That is to say, it can be said that by using these carriers for protein introduction it was possible to introduce β-galactosidase into the cells very efficiently.

### Working Example 2

The clay mineral was used to introduce β-galactosidase to the small intestine epithelial cells in vivo. It should be noted that as the carriers for protein introduction, the same carriers Sumecton SA (trade name) and Bentonite (trade name) as in Working Example 1 were used.

### 1. Complexing the Protein and the Carrier for Protein Introduction

50 µL of the carrier dispersion (1 mg/mL) and 50 µL of the protein dispersion (1 mg/mL), which were prepared in the same way as in the Working Example 1, were each diluted with 100 µL mili-Q water. The two dilute solutions were blended and incubated at 25°C for one hour to prepare protein introduction agents.

### 2. Protein Introduction in vivo

Using an oral sonde, the protein introduction agents forcibly were administered orally to 6-wk old ddY mice (male; Japan SLC) to a concentration of β-galactosidase 50 µg (45.25 U)/mouse. It should be noted that the mice were fasted from the day prior to administration of the protein introduction agents. As a control, 300 µL mill-Q water was administered in place of the protein introduction agent, and as a Comparative Example 2, a dilute solution obtained by diluting 50 µL of the protein dispersion (1 mg/mL) with 250 µL mili-Q water was administered orally in place of the protein introduction agent.

### 3. Verifying Protein Introduction

3 hours after oral administration, the mice were anesthetized with ether and their cervical vertebrae were dislocated. Then, the mice small intestine was removed and sectioned in 4 cm pieces from the stomach side for a total of three sections, and each of these then was immersed in 200 µL PBS (-) containing 5 mM EGTA. The small intestine was homogenized (Physcotron Homogenizer: made by NITI-ON) and centrifuged (14,500 rpm, 5 min, 4°C), and the supernatant liquid that was recovered was taken as the cell extract. The β-galactosidase activity of these cell extracts was measured in the same manner as in Working Example 1. The results for the cell extract from the section closest to the large intestine are shown in FIG. 2.

In FIG. 2, activity is seen in the control, but this is the β-galactosidase activity that was originally present in the cells. Thus, the difference between the control and the Working Examples 2 and the Comparative Example 2 can be determined to be the level of activity of due to the protein that was introduced. Accordingly, FIG. 2 shows that Working Examples 2-1 (Sumecton SA) and 2-2 (Bentonite) had much higher activity than Comparative Example 2. That is to say, by using these carriers for protein introduction, it was possible to introduce β-galactosidase very efficiently into cells of the small intestine in vivo as well. The fact that β-galactosidase is introduced in vivo and exhibits activity in a living organism indicates that an introduction agent that supports β-galactosidase can be used as a drug or a functional food product for lactose intolerance.

### Working Example 3

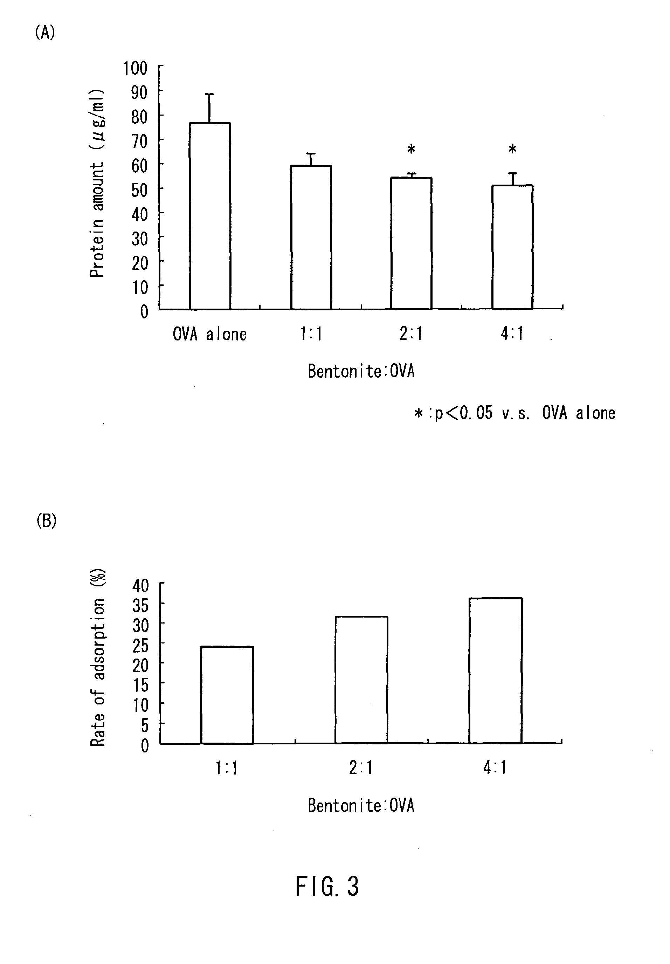

The adsorption of ovalbumin (OVA) to the clay mineral was confirmed. First, OVA and bentonite (trade name Bengel Fw; made by HOJUN) each were suspended in the PBS (-) of Working Example 1 to prepare an OVA dispersion (1 mg/mL) and a carrier dispersion (1 mg/mL). These dispersions were blended at the following blend ratio and incubated at room temperature for 1 hour and then centrifuged (4°C, 13,000 rpm, 30 min). The absorbance (260 nm, 280 nm) of the supernatant fraction was measured and the protein concentration was quantified (n=3). The results are shown in FIG. 3. FIG. 3(A) is a graph that shows the protein concentration of the supernatant, and FIG. 3(B) is a graph that shows the ratio of decrease in protein from OVA alone, where OVA alone is 100%, that is, it shows the rate of adsorption (%) to bentonite.

**Table 1**

| bentonite:OVA | PBS (µL) | 1 mg/mL bentonite (µL) | 1 mg/mL OVA (µL) |
|---|---|---|---|
| OVA alone | 180 | 0 | 20 |
| 1:1 | 160 | 20 | 20 |
| 2:1 | 140 | 40 | 20 |
| 4:1 | 100 | 80 | 20 |

The drawings confirm that the OVA was adsorbed to the bentonite by blending bentonite and OVA. The rate of adsorption of OVA was raised by increasing the amount of bentonite.

### Working Example 4

A protein introduction agent was prepared using a clay mineral and a protein that includes ovalbumin (OVA), which is the primary allergen of egg allergies, and the introduction of OVA to mice serum in vivo was confirmed.

### 1. Preparation of the Protein Introduction Agent

In the same manner as in Working Example 3, OVA and bentonite (trade name Bengel Fw) each were suspended in the PBS (-) of Working Example 1 to prepare an OVA dispersion (100 mg/mL) and a carrier dispersion (100 mg/mL). These dispersions were mixed at the following ratios within an Eppendorf tube and incubated at room temperature for 1 hour, thereby preparing protein introduction agents.

**Table 2**

| Protein Introduction Agent | PBS (-) (µL) | 100 mg/mL bentonite (µL) | 100 mg/mL OVA (µL) |
|---|---|---|---|
| control | 300 | 0 | 0 |
| Comparative Example 4: OVA alone | 250 | 0 | 50 |
| Working Example 4: bentonite-OVA complex | 150 | 100 | 50 |

### 2. Introduction of Protein in vivo

300 µL of the protein introduction agent was orally administered to 6-wk old ddY mice (male; Japan SLC) using an oral sonde (oral administration day 0, n=3). Further oral administration was conducted 7 days and 14 after the first administration. It should be noted that the mice were fasted from the day prior to administration of the protein introduction agents. As a control, 300 µL of the PBS (-) of Working Example 1 was administered in place of a protein introduction agent, and as a Comparative Example 4, 300 µL of the solution with OVA alone shows in Table 2 was administered orally in lieu of a protein introduction agent.

### 3. Verifying Protein Introduction

### (Preparation of a Serum Sample)

Blood samples were taken from the fundus oculi of the mice using a Pasteur pipette 14 days and 21 after the first oral administration. Immediately after taking the blood samples, the blood was centrifuged (4°C, 6,000 rpm, 5 min) and the blood serum was recovered. The serum sample was stored at -20°C until the assay described later.

### (ELISA Method)

The OVA-specific IgG antibody that is contained in the serum sample is allowed to act on a well plate coated with OVA, and the OVA-specific IgG antibody contained in the sample was detected using HRP-labeled antibody as a secondary antibody.

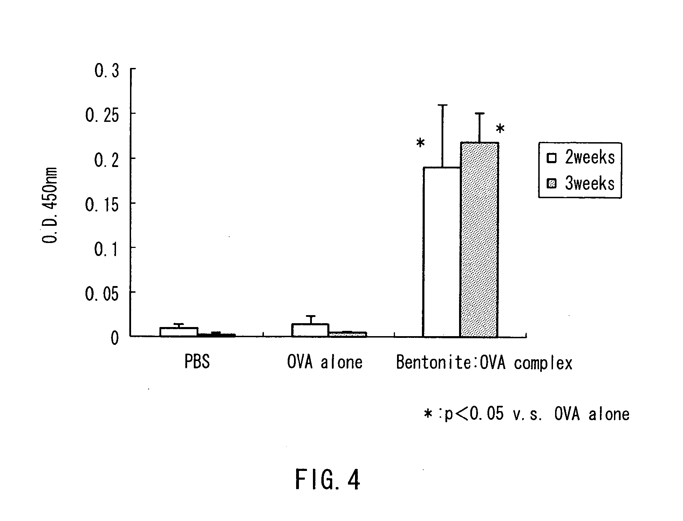

One day prior to the assay, the Coating Buffer listed below was added to the well plate until 100 µL/well, and then the well was covered and incubated at 4°C overnight. The next day, the well plate was washed three times with the Wash buffer listed below, and then the blocking buffer listed below was added until 300 µL/well. The well plate was incubated for 1 hour and then washed three times with the wash buffer. The serum sample was diluted by a dilution factor of 500 with the Sample diluent listed below, and this dilute sample was added to the well plate until 100 µL/well and then incubated for 2 hours. After incubation, the well plate was washed five times with the wash buffer. Next, the HRP detection antibody listed below and the sample diluent were blended at a blend ratio of 1:100,000, and this was added to the well plate until 100 µL/well and then incubated for one hour. The well plate was washed five times with the wash buffer and the TMB Solution described below was added until 100 µL/well and this was incubated for 30 minutes, and then 2M sulfuric acid was added until 100 µL/well and the absorbance was measured (450 nm, ref 595 nm). The results are shown in FIG. 4.

### (Methods for Preparing the Reagents)

Coating buffer: OVA is dissolved in 100 mM carbonate buffer (pH 9.6) until reaching an OVA concentration of 10 µg/mL.
Wash buffer: 50 mM Tris, 0.14 M NaCl and 0.05% Tween 20 (trade name) are blended together (pH 8.0).
Blocking buffer: Blocking one (trade name) and 50 mM Tris-HCl are blended to a 1:3 volume ratio.
Sample diluent: Blocking one (trade name) and the Wash solution are blended to a 1:19 volume ratio.
TMB solution: TMB solution (trade name) and Peroxidase solution (trade name) are blended immediately before use until 1:1 volume ratio.

As shown in FIG. 4, the model in which the OVA was administered alone (Comparative Example 4) had substantially the same results as the model in which only PBS was administered (control). It is presumed that this is because the antigen protein OVA is denatured or digested by the acidic conditions or the proteases in the stomach. In contrast to this, the model in which the protein introduction agent (bentonite-OVA complex) was administered (Working Example 4) demonstrates a significant increase in OVA-specific IgG in the serum compared to that of the results of the control and Comparative Example 4. This result indicates that the OVA has been introduced into the intestinal tract by the protein introduction agent of Working Example 4. That is to say, OVA forms a complex with the bentonite in the protein introduction agent of Working Example 4, and thus it is thought that when the OVA is introduced into the intestinal tract it is still protected from such damage in the stomach, and as a result elicits an immunological response from the intestinal tract and the increase in OVA-specific IgG in the serum is confirmed. The above results demonstrate that the carrier for protein introduction of the invention is for example useful as a protein delivery carrier in the oral treatment of hyposensitization against food allergies, for instance.

In Working Examples 5 through 7, the carrier for protein introduction is used and the introduction of a peptide and proteins in the range of 5 kDa to 500 kDa (for example, 6 to 465 kDa) is confirmed.

### Working Example 5

Using various carriers for protein introduction, an approximately 13 kDa molecular weight protein was introduced to the small intestine epithelial cell IEC-6 (ECACC-88071401) strain in vitro.

### 1. Complexing the Protein and the Carrier for Protein Introduction

Using four clay minerals as the carrier for protein introduction, these being Kunipia F (trade name) (made by Kunimine Industries), Bengel FW (trade name) (made by HOJUN), and Bengel Bright 25 (trade name) (made by HOJUN), which include montmorillonite, and Sumecton SA (trade name) (made by Kunimine Industries), which includes saponite, these were dispersed to 2 mg/mL in PBS (-) solution (Ca²⁺, Mg²⁺-free phosphate buffer saline solution: pH 7.2) to prepare carrier dispersions. On the other hand, midkine (hereinafter, also "MK") purchased from the PEPTIDE INSTITUTE, INC. was used as an example of the approximately 13 kDa molecular weight protein to complex with the carriers. The MK protein was dispersed in mili-Q water to 1 mg/mL to prepare a protein dispersion.

It should be noted that for the MK, it is also possible to produce a recombinant in an *E. coli* host, purify expressed MK and then use it as a sample in the experiment. Specifically, basically following the method of Take et al. (J. Biochem. 116:pp.1063-1068 (1994)), the cultured cell strain G-401 from a Wilm's tumor is used to prepare human MK cDNA with PCR by a standard method, and then the human MK cDNA is transformed to *E. coli* to create a recombinant. Then; the recombinant *E*. *coli* is grown through culturing, and from its inclusion body it is possible to purify the human MK protein according to the method of Take et al. in order to prepare the sample. It is also possible to ready the sample by purchasing a commercial product from the PEPTIDE INSTITUTE, INC. (Osaka) or R&D Systems (MN, USA).

Then, 15 µL of the carrier dispersion and 15 µL of the protein dispersion were added to and blended in 270 µL DMEM (Dullbecco's Modified Eagle's Medium: serum-free). The mixtures were incubated at room temperature for one hour, and then an additional 1 mL DMEM was added and this was mixed, producing protein introduction agents.

### 2. Introduction of Protein in vitro

As the cells, rat small intestine epithelial cells IEC-6 (ECACC-88071401) furnished from ATCC were used. First, 1.5 mL of the following liquid medium was added to a 12-well plate (made by Corning), and 5x10⁴ rat small intestine epithelial cells IEC-6 were inoculated in each well and cultured at 37°C for 4 days. It should be noted that all cells were cultured under an atmosphere of a 5% carbon dioxide concentration.

### (Composition of Liquid Medium)

DMEM (made by Invitrogen)
5 % FBS (fetal bovine serum: made by HyClone)
0.1 units/mL insulin (made by Invitrogen)
100 units/mL penicillin G sodium (made by Invitrogen)
100 µg/mL streptomycin (made by Invitrogen)
0.05 µg/mL amphotericin B (made by Dainippon Sumitomo Pharma)

After 4 days culturing, the cultured cells were washed twice with the PBS (-) solution, and 1.3 mL of the protein introduction agent was added to each well. Next, the wells were incubated at 37°C for 2 hours to introduce the protein (MK) into the cultured cells.

As a control, cells to which the protein introduction agent was not added, and as a Comparative Example 5, cells to which only MK was added, similarly were cultured. It should be noted that in Comparative Example 5, the 15 µL of protein dispersion was diluted by 1270 µL DMEM and 15 µL mili-Q water and added in place of the protein introduction agent.

After the incubation, all the cells were recovered. First, the cultured cells were washed twice with the PBS (-) solution. After washing, 100 µL of the sample processing solution listed below was added and the cells were recovered with a Cell Scraper and frozen at -80°C.

### (Sample Processing Solution Composition)

9 parts (specific volume) Tris SDS sample processing solution (made by Daiichi Pure Chemicals)
1 part (specific volume) 2-mercaptoethanol (made by Nacalai Tesque)
10 parts (specific volume) mili-Q water

### 3. Verifying Protein Introduction

The introduction of the protein to the rat small intestine epithelial cells was verified by Western blot analysis on the samples that were recovered and frozen in 2 above.

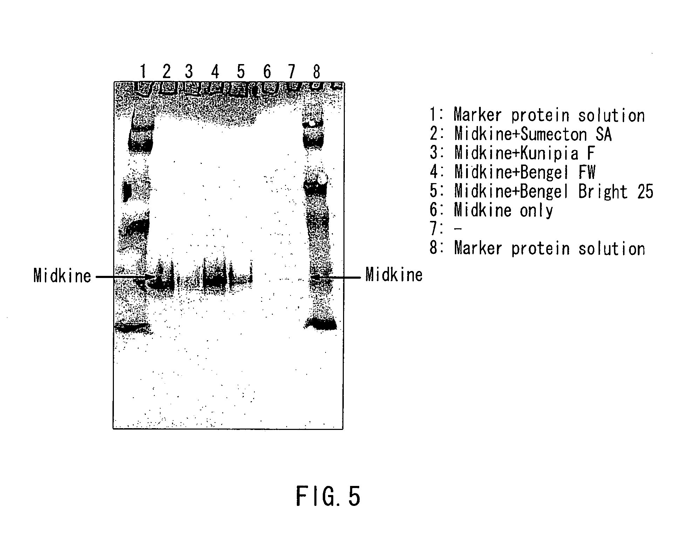

First, each sample was subjected to sodium dodecyl sulfate-polyacrylamide gel electrophoresis (SDS-PAGE) according to the method of Laemmli et al. As the focusing gel, PAG mini "Daiichi" 15/25 (trade name; Daiichi Pure Chemicals Co., Ltd.) was used, and the migration conditions were set to approximately 90 min at 20 mA constant current. Seven samples were applied to the wells, these being a marker protein solution, midkine+Sumecton SA, midkine+Kinipia F, midkine+Bengel FW, midkine+Bengel Bright 25, Comparative Example 5 (midkine only), and the control. The amount of sample per lane was the amount of protein of 2×10⁵ cells. A positive control was run adjusting chemically synthesized midkine (human) (PEPTIDE INSTITUTE, INC.) to 200 to 500 ng per lane (not shown in FIG. 5). The molecular marker that was used was (trade name) Kaleidoscope Prestained Standards (BIO-RAD).

Following the SDS-PAGE, a Western blot was performed according to an ordinary method. Immobilon (trade name) (MILLIPORE; MA, U.S.A.) was used for the blotting membrane, and transfer was conducted at 150 mA for 2 hours. After the transfer was finished, the membrane was slowly immersed overnight in the blocking solution (PBS solution that contains 4% skim milk and 0.05% Tween 20) while shaking, in order to suppress non-specific reactions. The membrane that had been blocked overnight was reacted with anti-human MK antibody (R&D Systems (MN, USA)) at room temperature for 2 hours. Following the reaction, the membrane was washed four times (10 min each) with a PBS solution that contains 0.05% Tween 20. The membrane then was reacted with donkey anti-goat IgG peroxidase labeled antibody (Santa Cruz Biotechnology, Inc.; CA, U.S.A.) at room temperature for 1 hour. Following the reaction, the membrane was washed four times (10 min each) with a PBS solution that contains 0.05% Tween 20, and then washed once (10 min) with a PBS solution.

The sufficiently washed membrane was reacted using a coloring kit (Immunostain HRP-1000 (trade name); Konica Minolta MG, Inc.). Several minutes after the start of the coloring reaction, a band was confirmed at the position of the MK molecular weight. FIG. 5 shows a photograph of a representative example of the result. In FIG. 5, lanes 1 and 8 are the result of marker protein solutions, lanes 2 through 5 are the result when a protein introduction agent is used, where lane 2 is midkine+Sumecton SA, lane 3 is midkine+Kunipia F, lane 4 is midkine+Bengel FW, and lane 5 is midkine+Bengel Bright 25, lane 6 is the result of Comparative Example 5 (midkine only), and lane 7 is the result of the control (-). From FIG. 5 it was confirmed that the band is darker when any one of the four protein introduction agents is used than when the protein is introduced alone (Comparative Example 5).

### Working Example 6

Using various carriers for protein introduction, an approximately 18 kDa molecular weight protein was introduced to the small intestine epithelial cell IEC-6 strain (ECACC-88071401) in vitro.

### 1 Complexing the Protein and the Carrier for Protein Introduction

As in Working Example 5, four clay minerals were used as the carrier for protein introduction, these being Kunipia F (trade name), Bengel FW (trade name), and Bengel Bright 25 (trade name), which include montmorillonite, and Sumecton SA (trade name), which includes saponite, to prepare the same carrier dispersions (2 mg/mL). On the other hand, pleiotrophin (hereinafter, also "PTN") purchased from the PEPTIDE INSTITUTE, INC. was used as an example of the approximately 18 kDa molecular weight protein to be complexed with the carriers. The PTN protein was dispersed in mili-Q water until 1 mg/mL to prepare a protein dispersion. It should be noted that for the PTN, like with MK, it is also possible to produce a recombinant and prepare recombinant PTN protein, and then use this as the sample, and it is also possible to use a commercial product from the PEPTIDE INSTITUTE, INC. (Osaka) or R&D Systems (MN, USA).

Then, 15 µL of a carrier dispersion and 15 µL of the protein dispersion were added to and blended in 270µL DMEM (Dullbecco's Modified Eagle's Medium: serum-free). The mixtures were left undisturbed for one hour at room temperature, and then an additional 1 mL DMEM was added and this was mixed, producing protein introduction agents.

### 2. Introduction of Protein in vitro

As the cells, rat small intestine epithelial cells IEC-6 (ECACC-88071401) furnished from ATCC were used. First, 1.5 mL of the same liquid culture medium as in Working Example 5 was added to a 12-well plate (made by Corning), and 5x10⁴ rat small intestine epithelial cells IEC-6 were inoculated to each well and cultured at 37°C for 4 days. It should be noted that all cells were cultured under an atmosphere of a 5% carbon dioxide concentration.

After 4 days culturing, the cultured cells were washed twice with the PBS (-) solution, and 1.3 mL protein introduction agent was added to each well. Next, the wells were incubated at 37°C for 2 hours and the protein (PTN) of the wells was introduced into the cultured cells.

As a control, cells to which the protein introduction agent was not added, and as a Comparative Example 6, cells to which only PTN was added, were cultured in the same manner. It should be noted that in Comparative Example 6, the 15 µL of carrier dispersion was diluted by 1270 µL DMEM and 15 µL mili-Q water and added in place of the protein introduction agent.

After the incubation, all the cells were recovered. First, the cultured cells were washed twice with the PBS (-) solution. After washing, 100 µL of the same sample processing solution as in Working Example 5 was added and the cells were recovered with a Cell Scraper and frozen at -80°C.

### 3. Verifying Protein Introduction

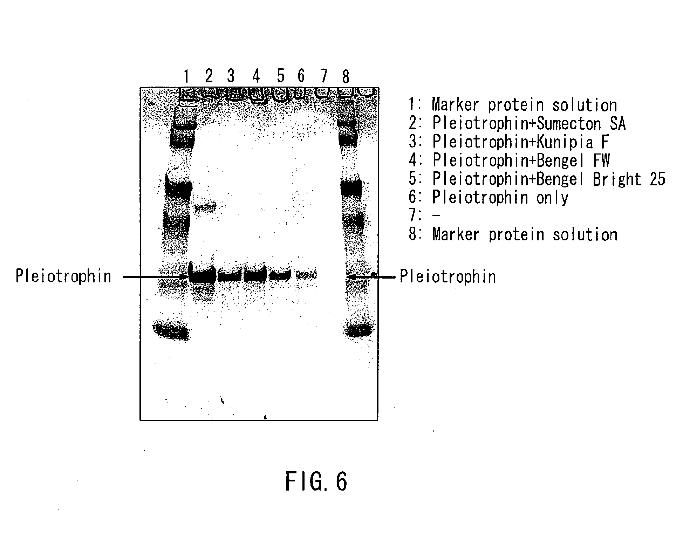

That the protein had been introduced into the rat small intestine epithelial cells was verified by performing Western blot analysis in the same manner as in Working Example 5, unless noted otherwise. It should be noted a positive control was run by adjusting chemically synthesized pleiotrophin (human) (PEPTIDE INSTITUTE, INC. (Osaka)) to 200 to 500 ng per lane (not shown in FIG. 6). The antibody that was used in the reaction with the membrane was anti-human PTN antibody (R&D Systems (MN, USA)). The results are shown in FIG. 6.

In FIG. 6, lanes 1 and 8 are the result of marker protein solutions, lanes 2 through 5 are the result when a protein introduction agent is used, where lane 2 is pleiotrophin+Sumecton SA, lane 3 is pleiotrophin+Kunipia F, lane 4 is pleiotrophin+Bengel FW, and lane 5 is pleiotrophin+Bengel Bright 25, lane 6 is the result of Comparative Example 6 (pleiotrophin only), and lane 7 is the result of the control (-). From FIG. 6 it was confirmed that the band is darker when any one of the four protein introduction agents is used than when the protein is introduced alone (Comparative Example 6).

### Working Example 7

Using various carriers for protein introduction, an approximately 5 kDa molecular weight polypeptide was introduced to the small intestine epithelial cell IEC-6 strain (ECACC-88071401) in vitro.

### 1. Complexing the Protein and the Carrier for Protein Introduction

The same clay mineral Sumecton SA (trade name), which includes saponite, as in Working Example 6 was used as the carrier for protein introduction, and the same carrier dispersion (2 mg/mL) was prepared. On the other hand, insulin (SIGMA-ALDRICH; MO, U.S.A.) (hereinafter, also "INS") was used as an example of an approximately 5 kDa molecular weight polypeptide to be complexed with the carrier. The INS was dispersed in a 5 mmol/L hydrochloric acid aqueous solution until 1 mg/mL in order to produce a polypeptide dispersion.

15 µL of the carrier dispersion and 15 µL of the polypeptide dispersion then were added to and mixed in 270 µL DMEM (Dullbecco's Modified Eagle's Medium: serum-free). This mixture was left undisturbed for one hour at room temperature, and then an additional 1 mL DMEM was added and this was mixed to produce a polypeptide introduction agent.

### 2. Introduction of Polypeptide in vitro

As the cells, rat small intestine epithelial cells IEC-6 (ECACC-88071401) furnished from ATCC were used. First, 1.5 mL of the same liquid culture medium as in Working Example 5 was added to a 12-well plate (made by Corning), and 5x10⁴ rat small intestine epithelial cells IEC-6 were inoculated to each well and cultured at 37°C for 4 days. It should be noted that all cells were cultured under an atmosphere with a 5% carbon dioxide concentration.

After 4 days culturing, the cultured cells were washed twice with the PBS (-) solution, and 1.3 mL of the polypeptide introduction agent was added to each well. Next, the wells were incubated at 37°C for 2 hours to introduce the INS into the cultured cells.

As a control, cells to which the protein introduction agent was not added was cultured in the same manner.

After the incubation, all the cells were recovered. First, the cultured cells were washed twice with the PBS (-) solution. After washing, 100 µL of the same sample processing solution as in Working Example 5 was added and the cells were recovered with a Cell Scraper and frozen at -80°C.

### 3. Verifying Protein Introduction

That the polypeptide had been introduced to the rat small intestine epithelial cells was verified by performing electrophoresis on the samples recovered and frozen in section 2.

First, each sample was subjected to sodium dodecyl sulfate-polyacrylamide gel electrophoresis (SDS-PAGE) according to the method of Laemmli et al. As the focusing gel, 18% Isocratic Gel (Invitrogen; CA, U.S.A.) was used, and the conditions for the migration were approximately 50 min at 50 mA constant current. Three samples were applied to the wells, these being a marker protein solution, insulin+Sumecton SA, and the control. The amount of sample per lane was the amount of protein of 2×10⁵ cells. A positive control was run by adjusting the insulin (SIGMA-ALDRICH; MO, U.S.A.) human) to 500 to 1000 ng per lane. The molecular marker that was used was (trade name) Kaleidoscope Prestained Standards (BIO-RAD).

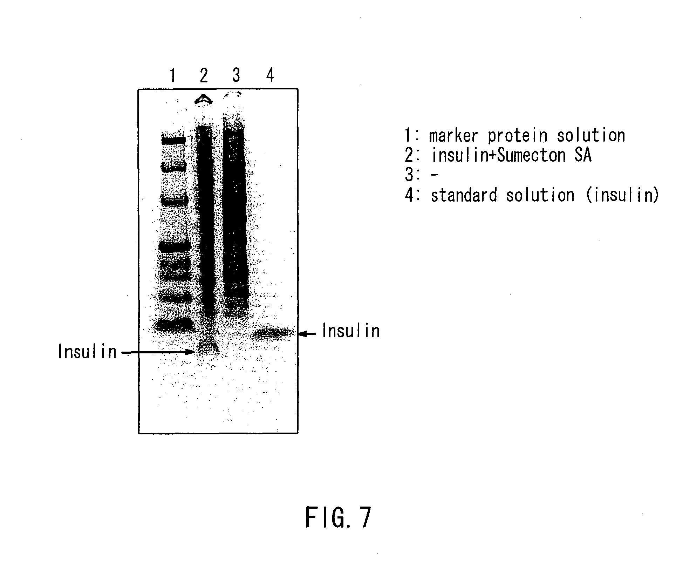

After the SDS-PAGE was finished, the gel was stained by Coomassie Brilliant Blue and the darkness of the insulin band in each lane was confirmed. FIG. 7 shows a photograph of a representative example of the result. In FIG. 7, lane 1 is the result of the marker protein solution, lane 2 is the result when the polypeptide introduction agent is used (insulin+Sumecton SA), lane 3 is the control (-), and land 4 is the result of a standard solution (positive control insulin). As shown in FIG. 7, a dark band that indicates that the insulin was introduced was confirmed when Sumecton SA, which includes saponite, was used.

### Industrial Applicability

In this way, using the carrier for protein introduction of the invention it is possible to very safely introduce a target protein into a cell. Thus, the method of protein introduction of the invention, which uses the carrier for protein introduction, is extremely useful in the field of clinical medicine.

## Claims

1. A carrier for protein introduction for introducing a protein to a cell, comprising a clay mineral.

2. The carrier for protein introduction according to claim 1,
wherein the clay mineral is a layered clay mineral.

3. The carrier for protein introduction according to claim 1,
wherein the clay mineral is at least one crystalline clay mineral selected from the group consisting of a kaolinite group, a pyrophyllite-talc group, a smectite group, a vermiculite group, a mica group, a brittle mica group, a chlorite group, and a sepiolite-paligoskite group.

4. The carrier for protein introduction according to claim 3,
wherein the clay mineral is at least one clay mineral selected from the group consisting of montmorillonite, vermiculite, and illite.

5. The carrier for protein introduction according to claim 1,
wherein the clay mineral is a clay mineral from which organic compounds have been removed.

6. The carrier for protein introduction according to claim 1,
wherein the clay mineral is a clay mineral that has been subjected to deionization.

7. The carrier for protein introduction according to claim 1,
wherein the clay mineral is at least 85% pure.

8. The carrier for protein introduction according to claim 2,
wherein the clay mineral has an exchangeable cation between its layers.

9. The carrier for protein introduction according to claim 8,
wherein the exchangeable cation is at least one cation selected from the group consisting of sodium ion, ammonium ion, and tertiary ammonium ion.

10. The carrier for protein introduction according to claim 8,
wherein the exchangeable cation is a cation of at least one substance selected from the group consisting of amino acids, amine compounds, amide compounds, cationic surfactants, and cationic metal complexes.

11. The carrier for protein introduction according to claim 1,
wherein the clay mineral supports the protein, and retains the protein under acidic conditions and releases the protein under neutral or alkaline conditions.

12. The carrier for protein introduction according to claim 1,
wherein the carrier is for introducing a protein to a cell in an organism.

13. The carrier for protein introduction according to claim 1,
wherein the carrier is for introducing a protein to a cell of the small intestine.

14. The carrier for protein introduction according to claim 1,
wherein the carrier is for oral administration.

15. The carrier for protein introduction according to claim 1,
wherein the carrier is for orally administrating a protein in an oral hyposensitization treatment.

16. The carrier for protein introduction according to claim 1,
wherein the carrier is for introducing a protein to a cultured cell.

17. A protein introduction agent that includes a carrier and a protein,
wherein the carrier is the carrier for protein introduction set forth in claim 1, and the protein is supported on the carrier for protein introduction.

18. The protein introduction agent according to claim 17,
wherein the blend ratio (mass ratio A:B) of the protein (A) and the clay mineral (B) contained in the carrier for protein introduction is in the range of 1:0.1 to 5.

19. The protein introduction agent according to claim 17,
wherein the protein introduction agent is in the form of a dispersion.

20. The protein introduction agent according to claim 19,
wherein the pH of the dispersion is in the range of 5.5 to 7.5.

21. A pharmaceutical composition that includes a protein as an agent for treating, preventing, or inhibiting a disease, further comprising:
the carrier for protein introduction that is set forth in claim 1.

22. The pharmaceutical composition according to claim 21,
wherein the pharmaceutical composition is for administration orally.

23. The pharmaceutical composition according to claim 21,
wherein the pharmaceutical composition is for hyposensitization treatment.

24. A protein introduction method of bringing a protein introduction agent that includes a carrier and a protein into contact with a cell in order to introduce the protein into the cell,
wherein the protein introduction agent is the protein introduction agent set forth in claim 17.

25. The protein introduction method according to claim 24,
wherein a ratio of the cells to the clay mineral contained in the protein introduction agent is in the range of 0.01 to 100 µg clay mineral per 1×10⁶ cells.

26. The protein introduction method according to claim 24,
wherein a ratio of the cells to the protein contained in the protein introduction agent is in the range of 0.01 to 100 µg protein per 1×10⁶ cells.

27. The protein introduction method according to claim 24,
wherein the cell is a cultured cell.

28. The protein introduction method according to claim 27, comprising:
incubating the cultured cell in advance of contact between the cultured cell and the protein introduction agent.

29. The protein introduction method according to claim 27,
wherein the protein introduction agent is contacted with the cultured cell for 3 to 48 hours.

30. The protein introduction method according to claim 24,
wherein the cell is a cell of an organism, and the protein introduction agent is administered into the organism and the protein is introduced into organ cells or tissue cells of the organism.

31. The protein introduction method according to claim 30,
wherein oral administration is the method by which the protein introduction agent is administered into the organism.

32. The protein introduction method according to claim 30,
wherein an organ to which the protein is introduced is the small intestine.

33. A method of producing protein-introduced cells by introducing an exogenous protein into cells,
wherein the method of introducing protein is the protein introduction method set forth in claim 24.

34. A protein-introduced cell to which an exogenous protein has been introduced,
wherein the protein-introduced cells are produced by the method set forth in claim 33.

35. A method of preventing or treating a disease by administering to a patient a pharmaceutical composition that includes a protein as an agent for treating, preventing, or inhibiting the disease,
wherein the pharmaceutical composition is the pharmaceutical composition set forth in claim 21.

36. The method of preventing or treating a disease according to claim 35,
wherein the method of administration is oral administration.

37. The method of preventing or treating a disease according to claim 35,
wherein the disease is diabetes and the protein is insulin.

38. The method of preventing or treating a disease according to claim 35,
wherein the prevention or treatment method is a method for hyposensitization treatment.

39. The method of preventing or treating a disease according to claim 38,
wherein the disease is an allergy and the protein is a protein responsible for the allergy.

40. Use of the carrier for protein introduction according to claim 1 in order to produce a pharmaceutical composition that includes a protein as an agent for treating preventing, or inhibiting a disease.

41. The use of the carrier for protein introduction according to claim 40,
wherein the pharmaceutical composition is a pharmaceutical composition that is to be administered orally.
